# EUROPEAN PATENT APPLICATION

(11) **EP 3 916 688 A1**
(43) Date of publication of application: **01.12.2021**
(21) Application number: 20757208.2
(22) Date of filing: 05.06.2020
(51) Int. Cl.: G07D 11/16, A61L 2/18, A61L 101/06, G07D 11/00, G07D 11/165

(54) **BANKNOTE PROCESSING APPARTUS HAVING DISINFECTION FUNCTION AND FINANCIAL DEVICE**

(30) Priority: 17.04.2020 CN 202010309855
(71) Applicant: Julong Co., Ltd, Anshan, Liaoning 114051 (CN)
(72) Inventor: NIU, Zuoqin, Anshan, Lianing 114051 (CN); YU, Miao, Anshan, Lianing 114051 (CN); MENG, Fangang, Anshan, Lianing 114051 (CN); GAO, Jitong, Anshan, Lianing 114051 (CN); YIN, Zhong, Anshan, Lianing 114051 (CN); LI, Weisi, Anshan, Lianing 114051 (CN); ZHANG, Yang, Anshan, Lianing 114051 (CN); LIU, Xuge, Anshan, Lianing 114051 (CN); WANG, Jiesheng, Anshan, Lianing 114051 (CN)
(74) Representative: Kolster Oy Ab
(86) International application number: PCT/CN2020/094739
(87) International publication number: WO 2021/208216

(57) **Abstract**

The present disclosure relates to the technical field of financial apparatus, and in particular to a banknote processing device having sterilization and disinfection functions and a financial apparatus. The banknote processing device having sterilization and disinfection functions comprises a banknote separation mechanism, a banknote transport mechanism, and a contact coating mechanism. While banknotes are being transported individually, the contact coating mechanism is in contact with a surface of a banknote, so that a sterilizing and disinfecting substance contained in the contact coating mechanism is adhered to the surface of the banknote, thus the banknotes can be disinfected one by one with a better disinfection effect. This solves the problem of poor disinfection effect caused by disinfecting banknotes in bundles in the prior art. Moreover, the disinfection by coating can ensure that the disinfectant solution can directly contact and adhere to the banknotes, and the disinfectant solution applied to the banknotes can fully disinfect the banknotes by permeation even while the banknotes are advancing at high a speed. This solves the problem in the prior art that banknotes cannot be effectively disinfected by ultraviolet irradiation, ozone disinfectant powder, high-temperature sterilization, or the like.

## Description

### Cross-Reference to Related Applications

The present disclosure claims priority of Chinese Patent Application No. 2020103098558, filed with the Chinese Patent Office on April 17, 2020, entitled "Banknote Processing Device Having Sterilization and Disinfection Functions and Financial Apparatus", the contents of which are incorporated herein by reference in their entirety.

### Technical Field

The present disclosure relates to the technical field of financial apparatuses, and in particular to a banknote processing device having sterilization and disinfection functions and a financial apparatus.

### Background Art

Banknotes to be disinfected are generally disinfected in form of bundles of banknotes by using disinfection cabinets in financial institutions. The disinfection method generally includes disinfection by ultraviolet radiation or by spraying of chemicals such as ozone disinfectant (sodium hypochlorite powder). However, since the above-mentioned disinfection operation is performed on an entire bundle of banknotes, the above-mentioned disinfection method has substantially no effect on the banknotes inside the bundle due to poor penetrability.

In order to improve the disinfection effect, the inventors of the present disclosure adds disinfection procedures to banknote processing apparatuses known in the art, such as counting and sorting machines, banknote counting machines, and automated teller machines, for disinfecting individual banknotes while counting the banknotes so as to solve the above-mentioned problem. Currently, the disinfection procedures added to the banknote processing apparatuses include ultraviolet radiation, ozone sterilization, high-temperature sterilization using heating rollers, etc.

However, banknotes are processed by the banknote processing apparatuses at rapid rates from a slow rate of 600 notes/minute to a fast rate of 900 notes/minute. When the processing rates are converted to the linear speeds of the banknotes inside the machines (apparatuses), it takes less than one millisecond for a banknote to advance per 1 mm. The widest banknote in the world has a width of 85 mm. In other words, it will not take more than 85 milliseconds for a banknote to pass under a heating roller or an ultraviolet lamp or a high-voltage electrode wire. However, the ultraviolet sterilization should generally be performed for 30 minutes before achieving its effect, while the high-temperature disinfection generally takes effect only after it is performed at a temperature higher than 100 degrees for a duration of more than 15 minutes. In addition, the high-voltage electrode wire and ozone have their own side effects, and they are also not disinfection means that can be effective in a short time. Therefore, none of the current disinfection methods in the banknote processing apparatuses has a substantively effect.

### Summary

An object of the present disclosure is to provide a banknote processing device having sterilization and disinfection functions and a financial apparatus to improve the effect of disinfecting banknotes by the banknote processing device.

The present disclosure provides a banknote processing device having sterilization and disinfection functions. The banknote processing device having sterilization and disinfection functions may comprise:
a banknote separation mechanism configured to separate multiple banknotes into individual ones and transport the banknotes to a banknote transport mechanism or to convey an individual banknote to the banknote transport mechanism;
the banknote transport mechanism configured to receive banknotes and transport the banknotes individually to a banknote reception area; and
a contact coating mechanism arranged on the banknote separation mechanism and/or the banknote transport mechanism;
optionally, while each banknote is being transported, the contact coating mechanism may be in contact with a surface of each banknote, so that a sterilizing and disinfecting substance contained in the contact coating mechanism is adhered to the surface of the banknote.

Optionally, the banknote transport mechanism may comprise a plurality of powered conveying units arranged in sequence; and each of the powered conveying units may comprise a first conveying part and a second conveying part disposed opposite to each other with a gap formed therebetween for conveying the banknotes;
the first conveying part may be a conveying roller, a conveying roller group, or a conveying belt; and the second conveying part may be a conveying roller, a conveying roller group, or a conveying belt;
the contact coating mechanism may comprise a coating member and a liquid supply member; and
the coating member may be disposed between any two of the powered conveying units, and the coating member has a coating surface that can contact a banknote being conveyed by the powered conveying units; the liquid supply member may be configured to supply the sterilizing and disinfecting substance to the coating member.

Optionally, each conveying roller is a powered roller, and the conveying roller is connected with a power mechanism to achieve the conveying function of the first conveying part and the second conveying part.

Optionally, the banknote transport mechanism may further comprise a banknote transport passage formed in a direction of conveying by the powered conveying units;
the first conveying parts and the second conveying parts partially extend into the banknote transport passage, so that the banknotes can be fed through the banknote transport passage;
the coating surface of the coating member extends into the banknote transport passage to be brought into contact with the banknotes in the banknote transport passage.

Optionally, one coating member is provided, and the coating member is disposed on one side of the banknote transport passage; or
a plurality of coating members are provided, and the plurality of coating members are distributed on one or both sides of the banknote transport passage.

Optionally, the banknote transport mechanism may further comprise an auxiliary roller;
the auxiliary roller may be disposed at a position opposite to at least one of the coating members with respect to the banknote transport passage, and the auxiliary roller partially extends into the banknote transport passage to assist in transporting of the banknotes in the banknote transport passage.

Optionally, the banknote separation mechanism may comprise a banknote receiving and dispensing wheel and a conveying passage, and the conveying passage communicates with a banknote inlet of the banknote transport mechanism;
the contact coating mechanism may comprise a coating member and a liquid supply member; and
the coating member has a coating surface extending into the conveying passage and in contact with the banknote being conveyed in the conveying passage; and the liquid supply member is configured to supply the sterilizing and disinfecting substance to the coating member.

Optionally, the banknote receiving and dispensing wheel extends into the conveying passage and is in contact with the banknotes in the conveying passage, a certain friction force is produced between the banknote receiving and dispensing wheel and each banknote, and the banknote receiving and dispensing wheel can rotate to drive the banknotes in the conveying passage to move along the conveying passage one by one.

Optionally, one coating member is provided, and the coating member is disposed on one side of the conveying passage; or
a plurality of coating members are provided, and the plurality of coating members are distributed on one side or both sides of the conveying passage.

Optionally, the banknote transport mechanism further comprises an auxiliary roller;
the auxiliary roller is disposed at a position corresponding to at least one of the coating members on an opposite side of the banknote conveying passage, and the auxiliary roller partially extends into the banknote conveying passage to assist in transporting of banknotes in the banknote conveying passage.

Optionally, the coating member may comprise a positioning member and a coating layer, and the positioning member may be arranged on the banknote separation mechanism and/or the banknote transport mechanism; and
the positioning member is in shape of a rod, the coating layer is provided on the positioning member, and the coating surface is formed on a side of the coating layer facing the banknote; or
the positioning member is in shape of a roller, the coating layer covers the positioning member, and the coating surface is formed by a surface of the coating layer on a side facing away from the positioning member.

Optionally, the coating layer is formed of a hydrophilic material.

Optionally, the coating layer has a length projected in a direction perpendicular to the banknote conveying direction that is no smaller than the length of each of the banknotes.

Optionally, the liquid supply member is a liquid storage member, and the coating layer partially extends into the liquid supply member; or
the liquid supply member is a spray member configured to spray the sterilizing and disinfecting substance to the coating layer.

Optionally, the contact coating mechanism may further comprise a liquid storage container, a liquid supply pump, and a liquid supply pipeline, wherein the liquid storage container is configured to store the sterilizing and disinfecting substance, and the sterilizing and disinfecting substance is pumped to the liquid supply member by the liquid supply pump and the liquid supply pipeline.

Optionally, the banknote processing device having sterilization and disinfection functions may further comprise:
a banknote detection mechanism configured to detect the authenticity, denomination, or quality of each of the banknotes; and/or
a banknote collection mechanism disposed in the banknote reception area.

The present disclosure further provides a financial apparatus, comprising a banknote processing device having sterilization and disinfection functions as described in any one of the above technical solutions.

Compared with the prior art, the present disclosure has the following advantageous effects:
The banknote processing device having sterilization and disinfection functions according to the present disclosure disinfects banknotes by coating using a contact coating mechanism while conveying the banknotes. On the one hand, the disinfection by coating is performed during the conveying of banknotes, thus the banknotes can be disinfected one by one with a better disinfection effect. This solves the problem of poor disinfection effect caused by disinfecting the banknotes in bundles in the prior art. On the other hand, the disinfection by coating can ensure that the disinfectant solution can directly contact and adhere to the banknotes, and the disinfectant solution applied to the banknotes can fully disinfect the banknotes by permeation even while the banknotes are advancing at a high speed. This solves the problem in the prior art that banknotes cannot be effectively disinfected by ultraviolet irradiation, ozone disinfectant powder, high-temperature sterilization, or the like.

The financial apparatus according to the present disclosure comprises the banknote processing device having sterilization and sterilization functions and hence also has the function of individually and fully disinfecting banknotes.

### Brief Description of Drawings

In order to more clearly illustrate technical solutions of specific embodiments of the present disclosure or of the technologies known by the inventors of the present disclosure, drawings required for use in the description of the specific embodiments or the technologies known by the inventors of the present disclosure will be described briefly below. It is obvious that the drawings in the following description are merely illustrative of some embodiments of the present disclosure and therefore should not be considered as limiting its scope. It will be understood by those of ordinary skill in the art that other drawings can also be obtained from these drawings without any inventive effort.
FIG. 1 is a schematic structural view of a banknote processing device having sterilization and disinfection functions according to an embodiment of the present disclosure;
FIG. 2 is another schematic structural view of a banknote processing device having sterilization and disinfection functions according to an embodiment of the present disclosure;
FIG. 3 is a first schematic structural view of a coating member arranged on a banknote transport mechanism according to an embodiment of the present disclosure;
FIG. 4 is a second schematic structural view of a coating member arranged on a banknote transport mechanism according to an embodiment of the present disclosure;
FIG. 5 is a third schematic structural view of a coating member arranged on a banknote transport mechanism according to an embodiment of the present disclosure;
FIG. 6 is a fourth schematic structural view of a coating member arranged on a banknote transport mechanism according to an embodiment of the present disclosure;
FIG. 7 is a fifth schematic structural view of a coating member arranged on a banknote transport mechanism according to an embodiment of the present disclosure;
FIG. 8 is a sixth schematic structural view of a coating member arranged on a banknote transport mechanism according to an embodiment of the present disclosure;
FIG. 9 is a seventh schematic structural view of a coating member arranged on a banknote transport mechanism according to an embodiment of the present disclosure;
FIG. 10 is an eighth schematic structural view of a coating member arranged on a banknote transport mechanism according to an embodiment of the present disclosure; and
FIG. 11 is a ninth schematic structural view of a coating member arranged on a banknote transport mechanism according to an embodiment of the present disclosure.

### Reference Numerals:

1-banknote separation mechanism, 11-banknote receiving and dispensing wheel, 12-conveying passage, 2-banknote transport mechanism, 21-first conveying part, 22-second conveying part, 23-banknote transport passage, 24-auxiliary roller, 25-banknote inlet, 31-coating member, 311-positioning member, 312-coating layer, 313-coating surface, 321-liquid storage member, 322-spray member, 33-liquid storage container, 34-liquid supply pump, 35-liquid supply pipeline, 4-banknote collection mechanism.

### Detailed Description of Embodiments

In order to further clarify the objects, technical solutions, and advantages of the embodiments of the present disclosure, the technical solutions of the present disclosure will be described below clearly and completely with reference to the drawings of the embodiments of the present disclosure. It is apparent that the embodiments to be described are some, but not all of the embodiments of the present disclosure.

Generally, the components of the embodiments of the present disclosure, as described and illustrated in the figures herein, may be arranged and designed in a wide variety of different configurations. Thus, the following detailed description of the embodiments of the present disclosure, as represented in the figures, is not intended to limit the scope of the present disclosure as claimed, but is merely representative of selected embodiments of the present disclosure.

All the other embodiments obtained by those of ordinary skill in the art in light of the embodiments of the present disclosure without inventive efforts will fall within the scope of the present disclosure as claimed.

In the description of the present disclosure, it should be noted that orientation or positional relations indicated by terms such as "center", "up", "down", "left", "right", "vertical", "horizontal", "inside", and "outside" are the orientation or positional relations shown based on the figures, and these terms are intended only to facilitate the description of the present disclosure and simplify the description, rather than indicate or imply that the referred devices or elements must be in a particular orientation or constructed or operated in the particular orientation, and therefore should not be construed as limitations on the present disclosure. In addition, the terms "first", "second", and "third" are used for descriptive purposes only, and should not be understood as an indication or implication of relative importance.

In the description of the present disclosure, it should be noted that the terms "mounted", "coupled", and "connected" should be understood broadly unless otherwise expressly specified or defined. For example, a connection may be fixed connection or detachable connection or integral connection, may be mechanical connection or electric connection, or may be direct coupling or indirect coupling via an intermediate medium or internal communication between two elements. The specific meanings of the above-mentioned terms in the present disclosure can be understood by those of ordinary skill in the art according to specific situations.

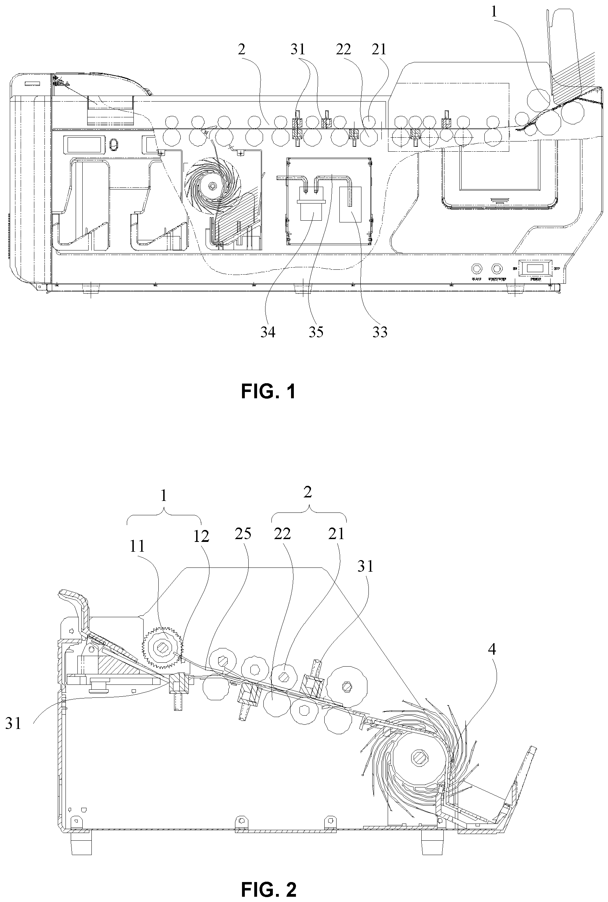

With reference to FIGS. 1 to 11, a banknote processing (or handling) device having sterilization and disinfection functions according to some embodiments of the present disclosure and a financial apparatus comprising the banknote processing device having sterilization and disinfection functions will be described below.

Referring to FIGS. 1 and 2, a banknote processing device having sterilization and disinfection functions according to an embodiment of the present disclosure comprises a banknote separation mechanism 1, a banknote transport mechanism 2, and a contact coating (applying) mechanism, wherein FIG. 1 shows an embodiment in which a banknote processing device is implemented as a horizontal multi-bin banknote counting and sorting machine, and FIG. 2 shows an embodiment in which a banknote processing device is implemented as a horizontal single-channel banknote counting and sorting machine. It should be noted that the banknote processing devices with sterilization and disinfection functions according to the embodiments of the present disclosure may be used as counting and sorting machines, banknote counting machines, automated teller machines and the like of other types or purposes. In the embodiments of the present disclosure, the banknote counting and sorting machines shown in FIGS. 1 and 2 are merely taken as an example for describing the specific structure and working principle of the banknote processing device.

Referring to FIGS. 1 and 2, the banknote separation mechanism 1 can transport an individual banknote to the banknote transport mechanism 2 or separate multiple banknotes into individual ones and transport the banknotes to the banknote transport mechanism 2. Optionally, the banknote separation mechanism 1 may comprise a banknote receiving and dispensing wheel 11 and a conveying passage 12. The conveying passage 12 may provide a space for movement of banknotes. The banknote receiving and dispensing wheel 11 may extend into the conveying passage 12 and be in contact with the banknotes in the conveying passage 12, and there may be a certain friction force between the banknote receiving and dispensing wheel 11 and the banknotes. Thus, when the banknote receiving and dispensing wheel 11 is rotating, the banknotes in the conveying passage 12 can be driven to move along the conveying passage 12 one by one, and the banknotes are conveyed to a banknote inlet 25 of the banknote transport mechanism 2 for subsequent transportation. Optionally, one or a plurality of banknote receiving and dispensing wheels 11 may be provided. When a plurality of banknote receiving and dispensing wheels 11 are provided, the plurality of banknote receiving and dispensing wheels 11 are arranged along the conveying direction in the conveying passage 12.

Referring to FIGS. 1 and 2, the banknote transport mechanism 2 can receive banknotes from the banknote separation mechanism 1 and transport the banknotes individually to a banknote reception area. The banknote transport mechanism 2 may comprise a plurality of powered conveying units arranged in sequence. The banknote inlet 25 of the banknote transport mechanism 2 is formed at one end of the plurality of powered conveying units that is close to the banknote separation mechanism 1, and a banknote outlet communicating with the banknote reception area is formed at the other end of the plurality of powered conveying units that is close to the banknote reception area. The plurality of powered conveying units cooperatively convey the banknotes in the conveying direction and transport the banknotes to the banknote reception area. Optionally, each of the powered conveying units may comprise a first conveying part 21 and a second conveying part 22 disposed opposite to each other with a gap formed therebetween. The gap between the first conveying part 21 and the second conveying part 22 may be configured to sandwich and convey a banknote. Optionally, the first conveying part 21 and the second conveying part 22 on both sides of a banknote may be in contact with the banknote during the conveying process to achieve the conveyance of the banknote, so that an individual banknote can be transported through the gap between the two conveying parts in the conveying direction.

Optionally, regarding the structures of the first conveying part 21 and the second conveying part 22, the first conveying part 21 and the second conveying part 22 may each be a conveying roller, a conveying roller group, or a conveying belt. Namely, the first conveying part 21 and the second conveying part 22 have different conveying manners, as long as they can drive a banknote to advance. Optionally, conveying rollers may be used as the first conveying part and the second conveying part, and both the conveying rollers may be powered rollers. Namely, each conveying roller may be connected with a power mechanism (e.g., a driving electric motor) to achieve the conveying function by the first conveying part and the second conveying part.

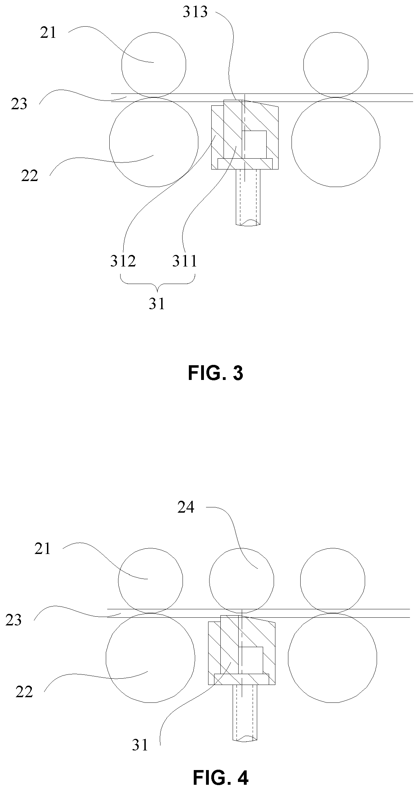

Optionally, referring to FIG. 3, the banknote transport mechanism 2 may further comprise a banknote transport passage 23 which is located between the first conveying parts 21 and the second conveying parts 22 and is formed in the direction of conveying by the powered conveying units. The banknote transport passage 23 communicates with the conveying passage 12. The first conveying parts 21 and the second conveying parts 22 partially extend into the banknote transport passage 23 so that banknotes can advance in the banknote transport passage 23. Optionally, the banknote transport mechanism 2 may comprise a conveying plate disposed along the banknote conveying direction, and the banknote transport passage 23 may be formed inside the conveying plate. The conveying plate is disposed such that a limit space for banknotes can be formed to ensure stable delivery of the banknotes in the conveying direction.

Optionally, the contact coating mechanism may be arranged on at least one of the banknote separation mechanism 1 and the banknote transport mechanism 2 to apply a disinfection treatment to individual banknotes while the banknotes are being conveyed individually in the banknote processing device of this embodiment. Namely, while the banknotes are being transported (in the banknote separation mechanism 1 and/or in the banknote transport mechanism 2), the coating member 31 may be in contact with a surface of each banknote, so that a sterilizing and disinfecting substance contained in the contact coating mechanism is adhered to the surface of the banknote.

Referring to FIG. 1 and FIGS. 3 to 11, the contact coating mechanism comprises a coating (applying) member 31, a liquid supply member, a liquid storage container 33, a liquid supply pump 34, and a liquid supply pipeline 35. The liquid supply pump 34 may be located between the liquid supply member and the liquid storage container 33, and the liquid storage container 33 and the liquid supply pump 34 may be connected with each other via the liquid supply pipeline and the liquid supply pump 34 and the liquid supply member may be connected with each other via the liquid supply pipeline 35 as well. Optionally, the coating member 31 may comprise a positioning member 311 and a coating layer 312 connected with the positioning member 311. The positioning member 311 may be used for mounting the coating member 31 to the banknote separation mechanism 1 or to the banknote transport mechanism 2 so that the coating layer 312 can contact a banknote being conveyed in the banknote separation mechanism 1 or in the banknote transport mechanism 2. A surface of the coating layer 312 which is in contact with the banknote may be a coating surface 313. Optionally, the coating layer 312 may be formed of a hydrophilic material (e.g., a sponge), which can absorb a certain amount of a disinfectant solution and apply the disinfectant solution to banknotes during the conveying of the banknotes. Optionally, the liquid storage container 33 may be configured to store the disinfectant solution, and the disinfectant solution in the liquid storage container 33 can be pumped to the liquid supply member by the liquid supply pump 34 and the liquid supply pipeline 35. The liquid supply member may be configured to supply the disinfectant solution to the coating layer 312, so that the disinfectant solution is always absorbed into the coating layer 312 to ensure the continuous proceeding of the disinfection of banknotes.

Optionally, a chlorine dioxide chemical solution may be selected and used as the disinfectant solution. As a disinfectant solution, the chlorine dioxide chemical solution is safe, efficient, and environmentally friendly, and thus for people, using the disinfected banknotes is also safer. Optionally, the liquid storage container 33 and the liquid supply pump 34 may be disposed in the banknote processing device or outside the banknote processing device.

The banknote processing device having sterilization and disinfection functions in this embodiment disinfects banknotes in a coating manner by a contact coating mechanism during conveying of the banknotes. On the one hand, the disinfection by coating is performed during the conveying of banknotes, thus the banknotes can be disinfected one by one with a better disinfection effect. This solves the problem of poor disinfection effect caused by disinfecting banknotes in bundles in the prior art. On the other hand, the disinfection by coating can ensured that the disinfectant solution can directly contact and adhere to the banknotes, and the disinfectant solution applied to the banknotes can fully disinfect the banknotes due to permeation even while the banknotes are advancing at a high speed. This solves the problem in the prior art that banknotes cannot be effectively disinfected by ultraviolet irradiation, ozone disinfectant powder, high-temperature sterilization or the like.

Referring to FIGS. 3 to 11, the specific structure of the contact coating mechanism arranged on the banknote transport mechanism 2 or the banknote separation mechanism 1 will be described below.

Optionally, when the contact coating mechanism is arranged on the banknote transport mechanism 2, the coating member 31 of the contact coating mechanism is disposed between any two of the powered conveying units of the banknote transport mechanism 2 or disposed on the powered conveying units.

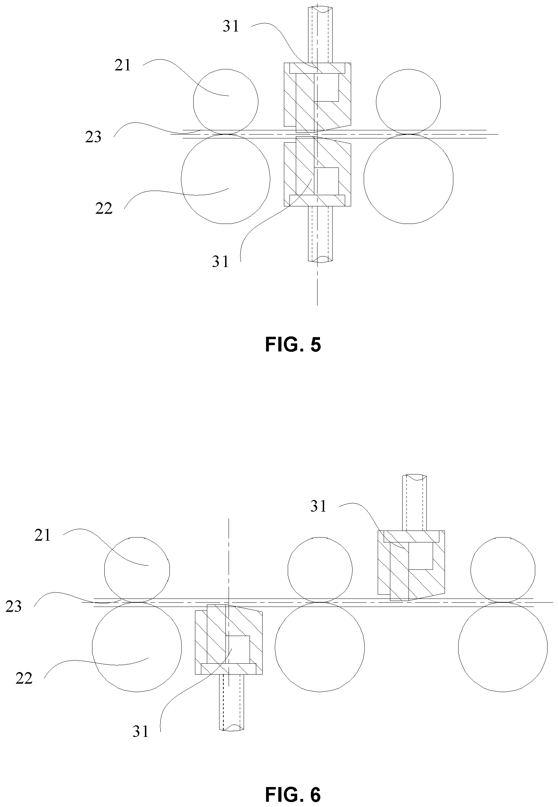

Referring to FIGS. 3 and 4, when the coating member 31 is disposed between any two of the powered conveying units of the banknote transport mechanism 2 and only one coating member 31 is provided, the coating member 31 is disposed on one side of the banknote transport passage 23. Referring to FIGS. 5 to 7, when the coating members 31 are disposed between any two of the powered conveying units of the banknote transport mechanism 2 and a plurality of the coating members 31 are provided, the plurality of coating members 31 may be distributed on one side or both sides of the banknote transport passage 23. Optionally, if the coating members 31 are all distributed on one side of the banknote transport passage 23, the coating members 31 may coat and disinfect only one side of each banknote during the movement of the banknotes in the banknote transport passage 23. If the plurality of coating members 31 are distributed on both sides of the banknote transport passage 23, the coating members 31 on both sides may coat and disinfect both sides of each banknote.

Referring to FIGS. 5 to 7, in the case where the plurality of coating members 31 are distributed on both sides of the banknote transport passage 23, the plurality of coating members 31 may be all distributed on both sides of the banknote transport passage 23 in a one-to-one correspondence manner, or may be partially distributed in a one-to-one correspondence manner and partially distributed in a staggered manner on both sides of the banknote transport passage 23, or may be all distributed in a staggered manner on both sides of the banknote transport passage 23.

Optionally, in the above distribution and arrangement cases of the coating members 31, except for the case where all of the plurality of coating members 31 are distributed on both sides of the banknote transport passage 23 in a one-to-one correspondence manner, no position-limiting structure is provided at a position on an opposite side to at least one coating member 31 (the position on the opposite side refers to a position located on the other side of the banknote transport passage 23 and symmetrical to the position of a coating member 31 with respect to the banknote transport passage 23 when the coating member 31 is disposed on one side of the banknote transport passage 23). Thus, referring to FIGS. 4, 7, 8, and 10, optionally, an auxiliary roller 24 may be disposed at the position on the opposite side to at least one coating member 31, in order to avoid the situation in which transporting of a banknote may be hindered or the banknote may be positionally deviated by large friction generated while coating the banknote by the coating members 31. Each auxiliary roller 24 may be a powered roller (power-driven rotatable) or a non-powered roller (rotatable without being power-driven). Optionally, the auxiliary roller 24 may partially extend into the banknote transport passage 23 and be in contact with banknotes to assist in transporting of the banknotes in the banknote transport passage 23.

Optionally, when the contact coating mechanism is arranged on the banknote separation mechanism 1 (not shown in the figures), the coating surface 313 of the coating member 31 extends into the conveying passage 12 of the banknote separation mechanism 1. One or more coating members 31 may also be provided, and the coating members 31 may also be distributed on one side or both sides of the conveying passage 12. An auxiliary roller 24 may also be disposed at the position on the opposite side to at least one coating member 31. The auxiliary roller 24 partially extends into the conveying passage 12 and is in contact with banknotes to assist in transporting of the banknotes in the banknote transport passage 23. Namely, the coating member(s) 31 may be distributed in the banknote separation mechanism 1 in a similar manner to that (those) distributed in the banknote transport mechanism, and therefore will not be described in detail here.

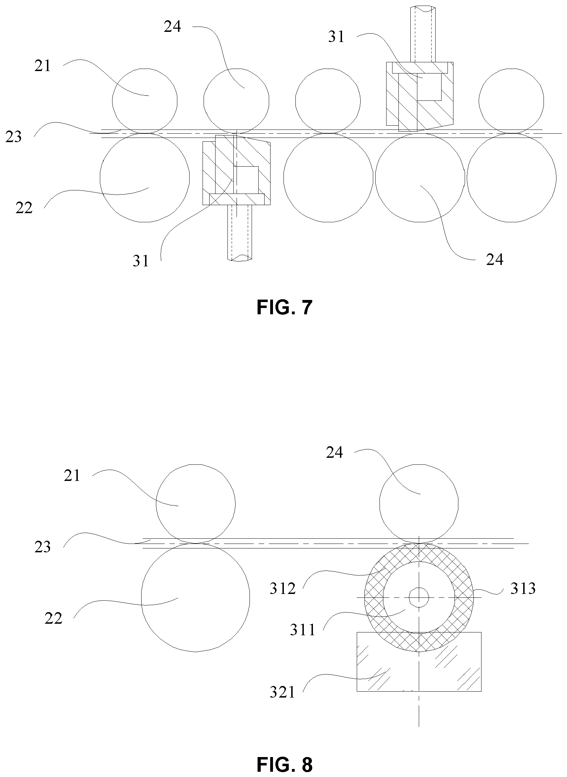

Optionally, the structures of each coating member 31 may include two specific structures. The specific structures of the coating member 31 will be described below with reference to FIGS. 3 and 8.

In the first case, as shown in FIG. 3, the positioning member 311 of the coating member 31 is in the shape of a rod and may be fixedly provided on the banknote transport mechanism (or the banknote separation mechanism 1) by a connector. Optionally, the coating layer 312 may be provided on the positioning member 311, and the coating surface 313 may be formed on a side of the coating layer 312 facing banknotes in the banknote transport passage 23 (or the conveying passage 12). Optionally, the coating member 31 of this structure does not move, and the banknotes are coated and disinfected by moving the banknotes and contacting the banknotes with the coating layer 312.

In the second case, as shown in FIG. 8, the positioning member 311 of the coating member 31 is in the shape of a roller. Namely, the coating member 31 is rotatable and may roll and convey in a direction consistent with the conveying of the banknotes. Moreover, the positioning member 311 may be fixedly provided on the banknote transport mechanism (or the banknote separation mechanism 1) by a connector. Optionally, the positioning member 311 may be entirely or partially covered by the coating layer 312 along the circumferential direction of the positioning member 311, and the coating surface 313 may be formed by a surface of the coating layer 312 at the side facing away from the positioning member 311. Optionally, the coating member 31 of this structure can rotate together with a banknote being conveyed while contacting the banknote, thereby reducing the resistance generated during the conveying of the banknotes and ensuring the stable delivery of the banknotes.

In the above-mentioned two structures, in order to ensure that the coating member 31 can apply coating on the entire surface of a banknote when coating the banknote, the coating layer 312 has a length projected in a direction perpendicular to the banknote conveying direction that is no smaller than the length of each of the banknotes,. Namely, a banknote can be wholly coated while the banknote is in contact with the coating member 31 regardless of whether the coating member 31 is disposed in a direction perpendicular to the banknote conveying direction.

Optionally, the structures of the liquid supply member may include two specific structures. The specific structures of the liquid supply member will be described below with reference to FIGS. 8 to 11.

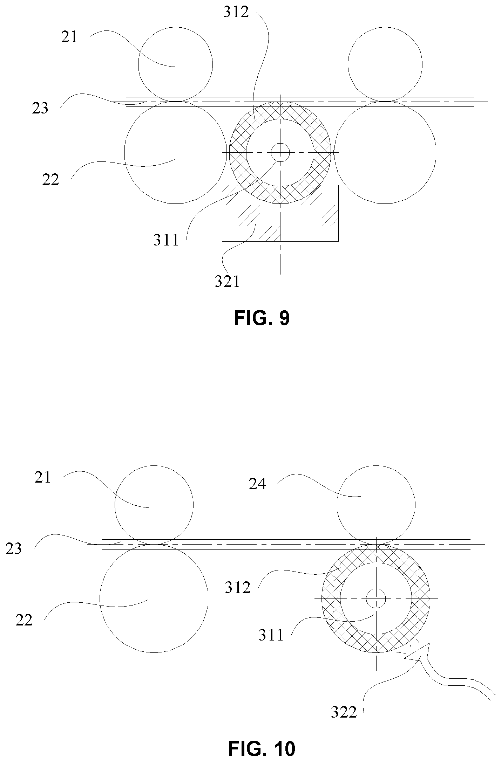

In the first case, as shown in FIGS. 8 and 9, the liquid supply member is a liquid storage member 321 (e.g., a liquid storage box) capable of storing a disinfectant solution, and the coating layer 312 partially extends into the liquid storage member 321, so that the liquid in the liquid storage member 321 is absorbed into the coating layer 312 by a liquid absorption effect of the coating layer 312, whereby the disinfectant solution is automatically supplemented into the coating layer 312.

Optionally, the liquid storage member 321 may be disposed under the coating layer 312. Optionally, the liquid storage member 321 may not be limited to being placed under the coating layer 312. When the coating member 31 is disposed above the banknote transport passage 23, the liquid storage member 321 may also be disposed above the coating layer 312 so as to facilitate supplying the liquid to the coating layer 312.

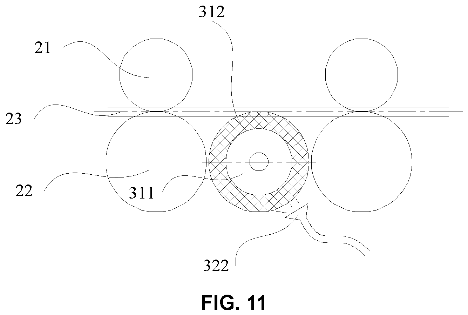

In the second case, as shown in FIGS. 10 and 11, the liquid supply member is a spray member 322 (e.g., a spray nozzle) capable of spraying or discharging a disinfectant solution to the coating layer 312. The disinfectant solution in the liquid storage container 33 can be pumped by the liquid supply pump 34 of the contact coating mechanism and then sprayed to the coating layer 312 by the spray member 322 so as to achieve supplying the disinfectant solution into the coating layer 312.

In addition, the banknote processing device having sterilization and disinfection functions in an embodiment of the present disclosure further comprises a banknote detection mechanism (not shown in the figures). The banknote detection mechanism may be disposed between the banknote separation mechanism 1 and the banknote transport mechanism. The banknote detection mechanism may be configured to identify, for example, the authenticity, denomination, and quality of each banknote. Optionally, a prior art banknote detection module may be used as the banknote detection mechanism, thus its specific structure will not be described in detail.

Furthermore, referring to FIG. 2, the banknote processing device having sterilization and disinfection functions according to an embodiment of the present disclosure further comprises a banknote collection mechanism 4. The banknote collection mechanism 4 is disposed at the banknote outlet of the banknote transport mechanism and is configured to receive conveyed and disinfected banknotes.

An embodiment of the present disclosure further provides a financial apparatus, comprising a banknote processing device having sterilization and disinfection functions according to any one of the embodiments described above. The financial apparatus comprises the banknote processing device having sterilization and disinfection functions and hence has all the advantageous effects of the banknote processing device, which will not be repeated here.

Finally, it should be noted that the above embodiments are merely intended to illustrate the technical solutions of the present disclosure, rather than limit the present disclosure. Although the present disclosure has been described in detail with reference to the foregoing embodiments, it should be understood by those of ordinary skill in the art that the technical solutions disclosed in the foregoing embodiments may still be modified, or some or all of the technical features thereof may be replaced with equivalents; and such modifications or replacements will not cause the essence of the corresponding technical solutions to depart from the scope of the technical solutions of the embodiments of the present disclosure.

### Industrial Applicability

The banknote processing device having sterilization and disinfection functions according to an embodiment of the present disclosure disinfects banknotes by coating using a contact coating mechanism while conveying the banknotes. On the one hand, the disinfection by coating is performed during the conveying of banknotes, thus the banknotes can be disinfected one by one with a better disinfection effect, which solves the problem of poor disinfection effect caused by disinfecting the banknotes in bundles in the prior art. On the other hand, the disinfection by coating can ensure that the disinfectant solution can directly contact and adhere to the banknotes, and the disinfectant solution applied to the banknotes can fully disinfect the banknotes due to permeation even while the banknotes are advancing at high speed, which solves the problem in the prior art that banknotes cannot be effectively disinfected by ultraviolet irradiation, ozone disinfectant powder, high-temperature sterilization, or the like.

## Claims

1. A banknote processing device having sterilization and disinfection functions, **characterized by** comprising:
a banknote separation mechanism configured to separate multiple banknotes into individual ones and transport the banknotes to a banknote transport mechanism or to convey an individual banknote to the banknote transport mechanism;
the banknote transport mechanism configured to receive banknotes and transport the banknotes individually to a banknote reception area; and
a contact coating mechanism arranged on the banknote separation mechanism and/or the banknote transport mechanism;
wherein while each banknotes is being transported, the contact coating mechanism is in contact with a surface of the each banknote, so that a sterilizing and disinfecting substance contained in the contact coating mechanism is adhered to the surface of the banknote.

2. The banknote processing device having sterilization and disinfection functions according to claim 1, wherein the banknote transport mechanism comprises a plurality of powered conveying units arranged in sequence; and each of the powered conveying units comprises a first conveying part and a second conveying part disposed opposite to each other with a gap formed therebetween for conveying the banknotes;
the first conveying part is a conveying roller, a conveying roller group, or a conveying belt; and the second conveying part is a conveying roller, a conveying roller group, or a conveying belt;
the contact coating mechanism comprises a coating member and a liquid supply member; and
the coating member is disposed between any two of the powered conveying units, and the coating member has a coating surface that can contact a banknote being conveyed by the powered conveying units; the liquid supply member is configured to supply the sterilizing and disinfecting substance to the coating member.

3. The banknote processing device having sterilization and disinfection functions according to claim 2, wherein each conveying roller is a powered roller, and the conveying roller is connected with a power mechanism to achieve the conveying function of the first conveying part and the second conveying part.

4. The banknote processing device having sterilization and disinfection functions according to any one of claims 2 and 3, wherein the banknote transport mechanism further comprises a banknote transport passage formed in a direction of conveying by the powered conveying units;
each of the first conveying part and the second conveying part partially extends into the banknote transport passage, so that the banknotes can advance in the banknote transport passage;
the coating surface of the coating member extends into the banknote transport passage to be in contact with the banknotes in the banknote transport passage.

5. The banknote processing device having sterilization and disinfection functions according to claim 4, wherein the banknote transport mechanism further comprises a conveying plate disposed along the banknote conveying direction, and the banknote transport passage is formed inside the conveying plate.

6. The banknote processing device having sterilization and disinfection functions according to any one of claims 4 and 5,
wherein one coating member is provided, and the coating member is disposed on one side of the banknote transport passage; or
a plurality of coating members are provided, and the plurality of coating members are distributed on one or both sides of the banknote transport passage.

7. The banknote processing device having sterilization and disinfection functions according to any one of claims 4 to 6, wherein the banknote transport mechanism further comprises an auxiliary roller;
the auxiliary roller is disposed at a position opposite to at least one of the coating members with respect to the banknote transport passage, and the auxiliary roller partially extends into the banknote transport passage to assist in advancing of the banknotes in the banknote transport passage.

8. The banknote processing device having sterilization and disinfection functions according to claim 1, wherein the banknote separation mechanism comprises a banknote receiving and dispensing wheel and a conveying passage, and the conveying passage communicates with a banknote inlet of the banknote transport mechanism;
the contact coating mechanism comprises a coating member and a liquid supply member; and
the coating member has a coating surface extending into the conveying passage and in contact with the banknote being conveyed in the conveying passage; and the liquid supply member is configured to supply the sterilizing and disinfecting substance to the coating member.

9. The banknote processing device having sterilization and disinfection functions according to claim 8, wherein the banknote receiving and dispensing wheel extends into the conveying passage and is in contact with the banknotes in the conveying passage, a certain friction force is generated between the banknote receiving and dispensing wheel and each banknote, and the banknote receiving and dispensing wheel can rotate to drive the banknotes in the conveying passage to move along the conveying passage one by one.

10. The banknote processing device having sterilization and disinfection functions according to any one of claims 8 and 9,
wherein one coating member is provided, and the coating member is disposed on one side of the conveying passage; or
a plurality of coating members are provided, and the plurality of coating members are distributed on one side or both sides of the conveying passage.

11. The banknote processing device having sterilization and disinfection functions according to any one of claims 8 to 10, wherein the banknote transport mechanism further comprises an auxiliary roller;
the auxiliary roller is disposed at a position opposite to at least one of the coating members with respect to the banknote conveying passage, and the auxiliary roller partially extends into the banknote conveying passage to assist in advancing of the banknotes in the banknote conveying passage.

12. The banknote processing device having sterilization and disinfection functions according to any one of claims 2 to 11,
wherein the coating member comprises a positioning member and a coating layer, and the positioning member is arranged on the banknote separation mechanism and/or the banknote transport mechanism; and
the positioning member is in shape of a rod, the coating layer is provided on the positioning member, and the coating surface is formed on a side of the coating layer facing the banknotes; or
the positioning member is in shape of a roller, the coating layer covers the positioning member, and the coating surface is formed by a surface of the coating layer on a side facing away from the positioning member.

13. The banknote processing device having sterilization and disinfection functions according to claim 12, wherein the coating layer is formed of a hydrophilic material.

14. The banknote processing device having sterilization and disinfection functions according to any one of claims 11 and 12,
wherein the coating layer has a length projected in a direction perpendicular to the banknote conveying direction that is no smaller than the length of each of the banknotes.

15. The banknote processing device having sterilization and disinfection functions according to any one of claims 12 to 14,
wherein the liquid supply member is a liquid storage member, and the coating layer partially extends into the liquid supply member; or
the liquid supply member is a spray member configured to spray a sterilizing and disinfecting substance to the coating layer.

16. The banknote processing device having sterilization and disinfection functions according to any one of claims 1 to 15, wherein the contact coating mechanism further comprises a liquid storage container, a liquid supply pump, and a liquid supply pipeline; the liquid storage container is configured to store the sterilizing and disinfecting substance, and the sterilizing and disinfecting substance is pumped to the liquid supply member by the liquid supply pump and the liquid supply pipeline.

17. The banknote processing device having sterilization and disinfection functions according to any one of claims 1 to 16, wherein the sterilizing and disinfecting substance is a chlorine dioxide chemical solution.

18. The banknote processing device having sterilization and disinfection functions according to any one of claims 1 to 17, further comprising:
a banknote detection mechanism configured to detect the authenticity, denomination, or quality of each of the banknotes; and/or
a banknote collection mechanism disposed in the banknote reception area.

19. A financial apparatus, **characterized by** comprising the banknote processing device having sterilization and disinfection functions according to any one of claims 1 to 18.
